# EUROPEAN PATENT SPECIFICATION

(11) **EP 4 761 810 B1**
(45) Date of publication and mention of the grant of the patent: **09.12.2026**
(21) Application number: 24762472.9
(22) Date of filing: 07.08.2024
(51) Int. Cl.: A61N 5/06

(54) **SYSTEMS AND METHODS FOR DETERMINING MINIMAL ERYTHEMA DOSE FOR PHOTOTHERAPY**
SYSTEME UND VERFAHREN ZUR BESTIMMUNG DER MINIMALEN ERYTHEMDOSIS FÜR DIE PHOTOTHERAPIE
SYSTÈMES ET PROCÉDÉS POUR DÉTERMINER UNE DOSE ÉRYTHÈME MINIMAL POUR UNE PHOTOTHÉRAPIE

(30) Priority: 07.08.2023 US 202363531290 P
(43) Date of publication of application: 24.06.2026
(73) Proprietor: Solius Labs, Inc., Bainbridge Island, WA 98110 (US)
(72) Inventor: GOOD, David, M., Bainbridge Island, WA 98110 (US)
(74) Representative: Forresters IP LLP
(86) International application number: PCT/US2024/041352
(87) International publication number: WO 2025/034913

(56) References cited:
- US-A1- 2005 143 793
- US-A1- 2012 039 827
- US-A1- 2013 231 720
- US-A1- 2015 238 774
- US-A1- 2020 376 292
- US-A1- 2022 383 496

## Description

### CROSS-REFERENCE TO RELATED APPLICATIONS

The present application claims priority to U.S. Provisional Patent Application No. 63/531,290, filed August 7, 2023.

### TECHNICAL FIELD

The present technology relates generally to phototherapeutic devices, systems, and methods. In particular, various embodiments of the present technology are related to systems and methods for determining a dosing protocol for phototherapy and associated devices.

### BACKGROUND

Vitamin D refers to a group of fat-soluble secosteriods that the human body can synthesize through adequate exposure to sunlight. More specifically, vitamin D3 is made in the skin when 7-dehydrocholesterol reacts with ultraviolet B ("UVB") light. Vitamin D can also be absorbed from the various dietary sources, such as fatty fish (e.g., salmon and tuna), vitamin D-fortified foods (e.g., dairy and juice products), and vitamin D supplements. Once absorbed, the vitamin D travels through the bloodstream to the liver where it is converted into the prohormone calcidiol. The calcidiol is, in turn, converted into calcitriol (the hormonally active form of vitamin D) by the kidneys or monocyte-macrophages in the immune system. When synthesized by the monocyte-macrophages, calcitriol acts locally as a cytokine to defend the body against microbial invaders. Kidney-synthesized calcitriol circulates through the body to regulate the concentration of calcium and phosphate in the bloodstream, and thereby promotes adequate mineralization, growth, and reconstruction of the bones. Therefore, an inadequate level of vitamin D, (typically characterized by a calcidiol concentration in the blood of less than 20-40 ng/m2) can cause various bone softening diseases, such as rickets in children and osteomalacia in adults. Vitamin D deficiency has also been linked to numerous other diseases and disorders, such as depression, heart disease, gout, autoimmune disorders, and a variety of different cancers.

Recently, vitamin D deficiency has become a prominent condition due, at least in part, to increasingly metropolitan populations and the resultant indoor lifestyles that inhibit adequate daily exposure to sunlight for vitamin D production. The growing emphasis on skin cancer awareness and sunscreen protection, which blocks UVB rays, may have also increased the spread of vitamin D deficiency. Additionally, various environmental factors, such as geographic latitude, seasons, and smog, further impede sufficient vitamin D production.

Physicians have recommended vitamin D supplements as a preventative measure to increase vitamin D levels. The American Institute of Medicine, for example, recommends a daily dietary vitamin D intake of 600 international units (IU) for those 1-70 years of age, and 800 IU for those 71 years of age and older. Other institutions have recommended both higher and lower daily vitamin D doses. The limitations on daily dosages also reflect an effort to prevent ingesting too much vitamin D, which can eventually become toxic. In contrast, the human physiology has adapted to significantly higher daily doses of vitamin D from sunlight (e.g., 4,000-20,000 IU/day or more). UVB radiation has been identified as a more desirable source of vitamin D because of the ease at which vitamin D is produced from exposure to sunlight and the body's natural ability to inhibit excessive vitamin D intake through the skin.
US2022383496A1 discloses a method for evaluating the effectiveness of a skin treatment on a skin feature includes correcting the color of a first image before the skin treatment, correcting the color of a second image after the skin treatment, determining the sizes of the skin feature in the first and second images, and comparing the corrected colors and sizes of the skin feature in the first and second images.
US2005143793A1 discloses an apparatus, system and method for the phototherapeutic treatment of psoriasis in which a change in at least one of color and texture is sensed as being indicative of erythema resulting from radiation.

### BRIEF DESCRIPTION OF THE DRAWINGS

Fig. 1A is an isometric front view of a phototherapy device configured in accordance with some embodiments of the present technology.
Fig. 1B is an isometric view of the phototherapy device of Fig. 1A positioned in a compact state in accordance with some embodiments of the present technology.
Fig. 2 is a flow diagram of a process for determining a dosing protocol for a user of a phototherapy device in accordance with some embodiments of the present technology.
Fig. 3A is a partially schematic side view of a phototherapy system while determining a dosing protocol for a user in accordance with some embodiments of the present technology.
Fig. 3B is a partially schematic side view of the phototherapy system of Fig. 3A while delivering a dose of UV radiation in accordance with some embodiments of the present technology.
Fig. 4 is an isometric view of a testing component for determining a dosing protocol for a phototherapy system configured in accordance with some embodiments of the present technology.
Fig. 5 is a flow diagram of a process for determining a dosing protocol using a testing component in accordance with some embodiments of the present technology.
Figs. 6A and 6B illustrate a result of the process of Fig. 5 for different users in accordance with some embodiments of the present technology.
Fig. 7 is an isometric view of a portion of a testing component of a phototherapy system configured in accordance with some embodiments of the present technology.

The drawings have not necessarily been drawn to scale. Similarly, some components and/or operations can be separated into different blocks or combined into a single block for the purpose of discussion of some of the implementations of the present technology. Moreover, while the technology is amenable to various modifications and alternative forms, specific implementations have been shown by way of example in the drawings and are described in detail below. The intention, however, is not to limit the technology to the particular implementations described.

### DETAILED DESCRIPTION

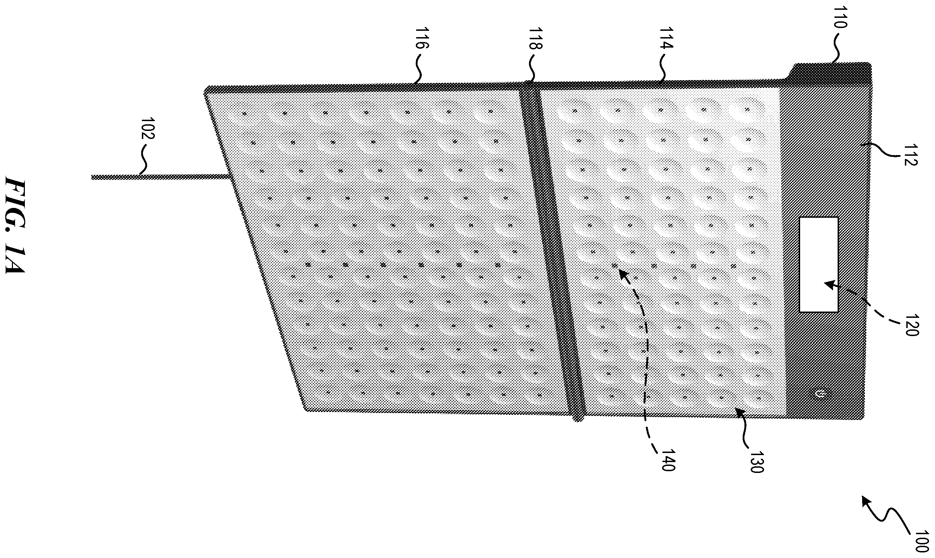

The present technology is directed to systems and methods for determining a dosing protocol for phototherapy and associated devices. Specifically, in a first aspect of the present invention there is provided a method for determining a dosing protocol for irradiation of a user's skin as set forth in the claims. In a further aspect of the present invention there is provided a phototherapy system as set forth in the claims. Specific details of several embodiments of the present technology are described herein with reference to Figures 1A-7. The present technology, however, can be practiced without some of these specific details. In some instances, well-known structures and techniques often associated with phototherapy, and the like, have not been shown in detail so as not to obscure the present technology. The terminology used in the description presented below is intended to be interpreted in its broadest reasonable manner, even though it is being used in conjunction with a detailed description of certain specific embodiments of the disclosure. Certain terms can even be emphasized below; however, any terminology intended to be interpreted in any restricted manner will be overtly and specifically defined as such in this Detailed Description section.

The accompanying Figures depict embodiments of the present technology and are not intended to be limiting of its scope. The sizes of various depicted elements are not necessarily drawn to scale, and these various elements can be arbitrarily enlarged to improve legibility. Component details can be abstracted in the Figures to exclude details such as position of components and certain precise connections between such components when such details are unnecessary for a complete understanding of how to make and use the present technology. Many of the details, dimensions, angles, and other features shown in the Figures are merely illustrative of particular embodiments of the disclosure. Accordingly, other embodiments can have other details, dimensions, angles, and features without departing from the present technology.

Phototherapy systems can provide users with exposure to UVB radiation to stimulate Vitamin D generation in the user's skin. In general, a suitable phototherapy system can include one or more sources of UV radiation (e.g., one or more UV lamps, one or more light-emitting diodes (LEDs), microplasma films, and/or the like) as well as one or more optical components to filter and shape the UV radiation (e.g., to emit only UV radiation in the UVB spectrum). Further, the phototherapy system can include a dose controller that helps regulate the amount of UV radiation the phototherapy system provides. The dose controller can set the intensity and/or duration of a dose delivered to the user, track doses, confirm whether the user experienced erythema after a previous dose, and/or automatically adjust doses over time as the user builds a tolerance to the UV radiation and/or drops in tolerance (e.g., after experiencing an erythema event, not dosing for an extended period, and/or the like).

The Fitzpatrick skin type has been used to set an initial dose for a phototherapy treatment. The Fitzpatrick skin type scale is a numerical classification schema for human skin color as a way to estimate how a user will respond to UV radiation and/or how much UV radiation a user can tolerate. For example, a user's response to a number of questions regarding the shade of the user's skin, hair color, eye color, propensity to burn, and the like, can be used to assign the user to one of six skin types (Type I-Type VI). The user's skin type can then be used to estimate the minimal erythema dose ("MED") for a user. A user's MED is the UV dose that produces perceptible erythema in the user's skin 24 hours after exposure.

Because determining skin type based on a user's assessment of subjective questions (e.g., how does your skin react to 1 hour of sunlight), the question-based Fitzpatrick assessment can result in inaccuracies. Furthermore, the Fitzpatrick skin type scale does not account for variations within a skin type, which can affect the skin's response to UV radiation. For example, a user who is normally relatively sensitive to UV radiation but has acquired a suntan from repeated phototherapy exposures and/or sun exposure will have developed a relatively high tolerance for their skin type and therefore require more UV radiation to reach their MED. In another example, a user with a pre-existing sunburn will require less radiation (or no radiation) to reach their MED.

Due to some limitations associated with the Fitzpatrick skin type scale assessment, previous vitamin-D phototherapy systems frequently estimated a user's MED based on their skin type, set an initial dosing protocol well below the user's MED, and slowly ramped up radiation exposure in each session toward the estimated MED, waiting a predefined time (e.g., 1 week) between each exposure. While the process provides a safe ramp-up in the amount of UV exposure to reduce or eliminate the likelihood of any UV exposure burn, the slow process meant that users might not experience the full benefits of the phototherapy for 6-8 weeks. Further, if a user interrupted their treatments (e.g., stopped receiving treatments for a month), their tolerance to UV radiation would typically drop, requiring the process to reduce the UV dose and ramp back up again.

Phototherapy devices and associated systems and methods are disclosed herein are expected to provide accurate skin color assessments and do so within a short period of time (e.g., within seconds, minutes, hours, 24 hours, and/or any time therebetween). This allows the associated phototherapy system to set appropriate exposure parameters for a dose of phototherapy below or at a user's MED before each phototherapy session and ensures appropriate exposure to receive the positive effects of the phototherapy (e.g., vitamin D production) at each session, without needing an elongated calibration period.

In some embodiments, the phototherapy devices can include a color testing component (e.g., a colorimeter) that obtains images (or other measurements) of a user's skin. The phototherapy devices (or another device communicatively coupled thereto, such as a remote server) can use convert the images to an L*a*b* format (sometimes also referred to as CIELAB color format), which provides an accurate measurement of all perceivable colors in a treatment region of the user's skin. The letters L*, a*, and b* represent each of the three values the CIELAB color space uses to measure objective color and calculate color differences. L* represents lightness from black to white on a scale of 0 to 100, while a* and b* represent chromaticity with no specific numeric limits. Negative a* typically corresponds to green colors, positive a* typically corresponds to red colors, negative b* typically corresponds to blue colors, and positive b* typically corresponds to yellow colors.

The L*a*b* format is especially useful because the values have been correlated with the individual topography angle (ITA) of a user's skin. ITA is a measure of skin pigmentation related to the amount of light absorbed by melanin in a user's skin. The ITA can be used to classify the user's skin type within one of six broad categories that are associated with how much UV radiation a user can tolerate. However, ITA values provide a continuous spectrum of values, expressed as angles, within the categories that can then be used to calculate a MED for the user.

Because ITA values are obtained from an objective color measurement of the user's skin, the ITA values can provide a more accurate method to calculate a user's MED. As a result, a phototherapy device using ITA to estimate a user's MED and/or determine a dosing protocol does not need to ramp up to an appropriate dose. Instead, the phototherapy device can provide a full dose to the user the same day (sometimes within minutes) of measuring the user's skin, thereby allowing users to more quickly experience the full benefits of the phototherapy device. Further, because the ITA is based on images of a user's skin, the ITA value is immediately responsive to changes in the user's skin (e.g., the development of a suntan and/or sunburn). As a result, the phototherapy device can quickly adapt to the user as they build a tolerance and/or accurately reduce a dosing protocol after a long period of no exposures (e.g., associated with a loss of a suntan). Still further, because ITA values are expressed on a continuous spectrum, rather than in six discrete skin types, the MED and/or dosing protocol calculated from an ITA value can be more granularly tailored to specifics in the user's skin.

In some embodiments, the phototherapy systems disclosed herein can use a strip testing component (also referred to as a "zap test") in addition to, or in alternative to, a color testing component. The strip testing component can deliver a plurality of incrementally increasing doses of UV radiation to a user's skin in discrete locations. The incremental increases can help identify the user's skin type and/or an appropriate dose within a user's skin type through direct exposure to different levels of UV radiation. A predetermined time period (e.g., 24 hours) after the strip test, the user, another person (e.g., a medical professional), and/or an imaging component can assess the discrete locations to determine the number of erythema events the user experienced and/or the largest dose that did not result in an erythema event. The phototherapy system can then use this information to set a dosing protocol for the user. While the strip testing method may cause some UV-induced pigmentation of a user's skin to identify an appropriate dose of UV radiation, the discrete locations tested are relatively small (e.g., 1 centimeter (cm) in diameter, 2.6 cm in diameter, less than 2.6 cm in diameter, less than 1 cm in diameter) in comparison to the treatment region and, therefore, allow the user to avoid UV-induced pigmentation across their entire treatment region. Additionally, the strip testing method can identify the appropriate dose of UV radiation 24 hours after testing, rather than requiring weeks to ramp up. As a result, the user can begin experiencing the full benefits of the phototherapy 24 hours after their initial test.

For ease of reference, the phototherapy device, components thereof, and testing devices are sometimes described herein with reference to top and bottom, upper and lower, upwards and downwards, and/or horizontal plane, x-y plane, vertical, or z-direction relative to the spatial orientation of the embodiments shown in the figures. It is to be understood, however, that the phototherapy device, components thereof, and testing devices can be moved to, and used in, different spatial orientations without changing the structure and/or function of the disclosed embodiments of the present technology.

Further, although primarily discussed herein as a determining a user's MED and/or dosing protocol for phototherapy systems, one of skill in the art will understand that the scope of the technology is not so limited. For example, the systems and methods disclosed here can also be used to establish a user's ITA, MED, and/or UV dose tolerance for other medical treatments. Accordingly, the scope of the technology is not confined to any subset of embodiments disclosed herein.

### Selected Embodiments of Phototherapy Devices Using ITA and Related Systems and Methods

Fig. 1A is an isometric view of a phototherapy device 100 configured in accordance with some embodiments of the present technology. As illustrated in Fig. 1A, the phototherapy device 100 ("device 100") includes a housing 110 that has a header portion 112, a first panel 114, and a second panel 116 coupled to the first panel 114 by a hinge 118. The header portion 112 can carry various control electronics 120 (e.g., a controller and/or various other suitable electronics) while the first and second panels 114, 116 can carry an array of UV emitters 130 and/or a color testing component 140.

The control electronics 120 can include a non-transitory memory and a processor, a communication component, and/or the like. The memory and processor can implement any of the processes discussed herein to control components of device 100 (e.g., the array of UV emitters 130, the color testing component 140, the communication component, and/or the like), communicate with another device (e.g., a personal electronic device associated with a user, a remote server, and/or the like), and/or the like. In some embodiments, the control electronics 120 include one or more input devices, such as a touchscreen, keypad, and the like that allow a user to interact directly with the device 100 (e.g., instead of through another electronic device, such as their smartphone) to turn the device 100 on, set a dosing protocol, receive a dose of UV radiation, turn the device 100 off, and the like. Additionally, or alternatively, the input devices can include various audio, video, and/or biometric sensors that can help authenticate a user to quickly retrieve a dosing protocol, treatment history, medical data, and/or the like.

The array of UV emitters 130 is configured to emit UV radiation toward a user of the device 100 with a uniform (or generally uniform) density. For example, the array of UV emitters 130 can include an array of light-emitting diodes (LEDs) that emit UV radiation and one or more optical components to expand, collimate, and/or filter the UV radiation from the LEDs. In various embodiments, the LEDs can emit UV radiation with a wavelength between about 285 nanometers (nm) to about 315 nm, between about 293 nm to about 305 nm, or of about 297 nm. The optical components can then expand and collimate the beams from the LEDs to create a uniform (or generally uniform) density in the UV radiation emitted from the device 100. The uniform (or generally uniform) density can help improve the consistency of UV radiation a user receives from the device 100. The consistency, in turn, helps reduce the number of spots that receive too much UV radiation (e.g., and thereby experience localized erythema). Additionally, or alternatively, the optical components can filter the UV radiation to remove radiation outside of a predetermined range, above a predetermined threshold, and/or below a predetermined threshold. Vitamin D production has been shown to be maximized by UV radiation with specific wavelengths (e.g., wavelengths around 297 nm). Accordingly, by reducing the UV radiation outside of a desired range (e.g., between about 285 nm and about 305 nm) the filter can reduce a user's overall exposure to UV radiation without reducing the Vitamin D-related effects. Additional details of examples of suitable components for the array of UV emitters 130 are disclosed in U.S. Application No. 18/021,922 to Gary Lauder et. al filed February 17, 2023.

The color testing component 140 is positioned to measure various parameters of the user's skin (e.g., melanin in the user's skin) when they stand in front of the first and second panels 114, 116. For example, the color testing component 140 can include a colorimeter, light emitting device with an RGB sensor or other imaging sensor, and/or any other suitable component that takes one or more measurements of the user's skin (referred to herein as "images" of the user's skin). The images can include L*a*b* data, chroma data, hue data, and/or RGB data related to how the user's skin absorbs (and reflects) various wavelengths of light. As discussed in more detail below, the color testing component 140 and/or the control electronics 120 can use the images to calculate an individual topography angle (ITA) for the user, calculate a minimal erythema dose (MED) and/or a dosing protocol based on the ITA, detect erythema in the user's skin, melanin in the user's skin, and/or the like.

As further illustrated in Fig. 1A, the device 100 can also include an external power supply 102 (e.g., a power cord). The external power supply 102 can simplify and/or stabilize the operation of the device 100 by accessing a consistent power source (e.g., avoiding drops in the actual UV radiation delivered when a battery runs low). However, in some embodiments, the device 100 is powered by an internal power component (e.g., an onboard battery) that can be recharged and/or swapped as needed. The internal power component can increase a portability of the device 100 by reducing a reliance of the device 100 on access to power during use.

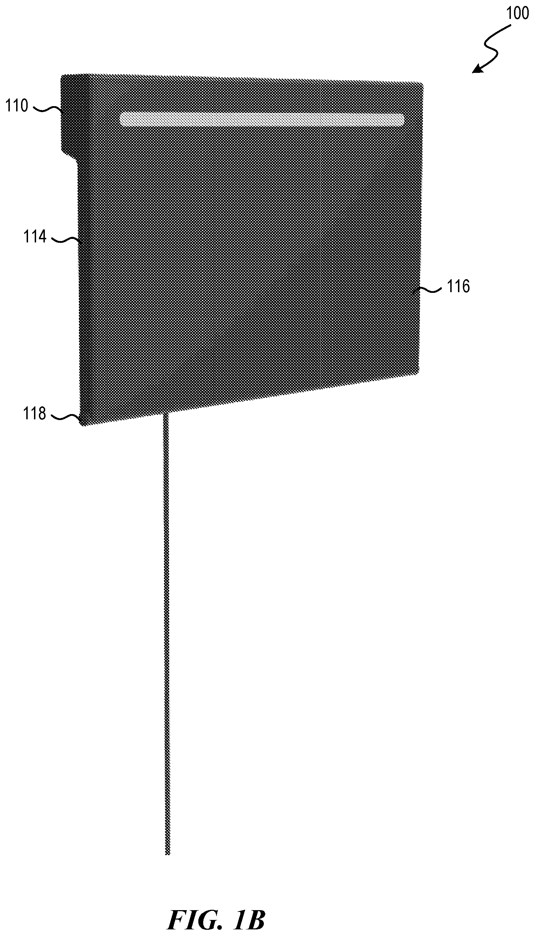

Fig. 1B is an isometric view of the device 100 of Fig. 1A in a compact state (also referred to as a folded state, collapsed state, stowed state, or the like) in accordance with some embodiments of the present technology. As illustrated in Fig. 1B, the second panel 116 can be folded into contact with the first panel 114 via the hinge 118. As a result, the array of UV emitters 130 on the first and second panels 114, 116 are covered, which can help reduce the chance of accidental exposures from the device 100.

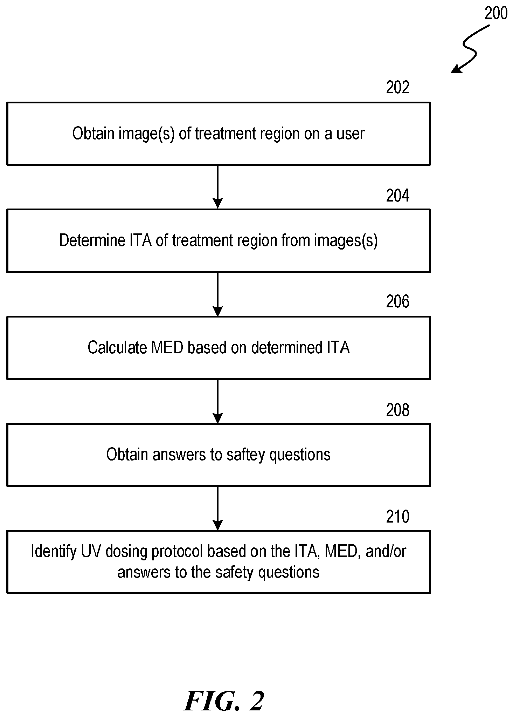

Fig. 2 is a flow diagram of a process 200 for determining a dosing protocol for a user of a phototherapy device in accordance with some embodiments of the present technology. The process 200 can be implemented by the control electronics 120 of Fig. 1A and/or any other suitable controller (e.g., a remote server coupled to the control electronics 120).

The process 200 begins at block 202 by obtaining (e.g., capturing, receiving, and/or the like) one or more images of a treatment region on a user. In some embodiments, obtaining the images at block 202 includes operating a colorimeter to emit light with a known composition of wavelengths (e.g., white light LEDs) and measure a reflection back from the treatment region using an imaging sensor (e.g., an RBG sensor and/or another suitable sensor). In some embodiments, obtaining the images at block 202 includes imaging the treatment region and a calibration component sequentially and/or at the same time. The calibration component can have a known color (e.g., a known yellow hue), allowing the imaging sensor (or any suitable processor) to account for ambient light. For example, the process 200 at block 202 can determine a corrective filter based on images of the calibration component that account for the known color, then apply the corrective filter to one or more pre-filtered images of the treatment region. In some embodiments, obtaining the images at block 202 includes receiving the images from a separate component (e.g., from a user's smartphone).

At block 204, the process 200 includes determining the ITA of the treatment region from the images. Determining the ITA can include converting the images to L*a*b* format (sometimes also referred to as CIELAB color format) where L* represents perceptual lightness on a scale from 0 (black) to 100 (white), a* represents a green-magenta color axis on a negative/positive scale (typically from -128 to +127), and b* represents a blue-yellow color axis on a negative/positive scale (typically from -128 to +127). The ITA can be calculated from L*a*b* data using: $ITA = \frac{arctan \left(\frac{L * - 50}{b *}\right) \times 180}{π}$ (1) In some embodiments, the L*a*b* data is averaged over the treatment region before calculating a single ITA. In some embodiments, multiple ITAs are calculated (e.g., for each pixel in an image of the treatment region), then averaged to determine a final ITA. The averages can account for changes in pigment across the treatment region, localized areas of high UV sensitivity (e.g., small sunburns), overall UV sensitivity (e.g., accounting for suntans), and/or the like. As a result, the averages allow the process 200 to determine an ITA that can be more accurate to the entire treatment region than a single point sampled from the treatment region.

At block 206, the process 200 calculates the user's estimated MED based on the ITA determined at block 204. The estimated MED can be calculated using: $MED = \left({ITA}^{2}\times 0.051\right) - \left(ITA\times 10.718\right) + 629.32$ (2) In some embodiments, the process 200 determines a plurality of ITAs at block 204 (e.g., the ITA at each pixel in the image of the treatment region), and then calculates a plurality of MEDs at block 206. The process 200 can then calculate a final MED by averaging the MEDs that are calculated using the plurality of ITAs.

At block 208, the process 200 includes receiving and/or obtaining answers to one or more secondary (e.g., safety) questions. The secondary questions can include whether the user is taking any medications, which may raise or lower the user's actual MED despite their ITA; whether the user has recently been exposed to sunlight (or other UV radiation), which can supply a portion of the UV to reach the user's MED; whether the user has a particular susceptibility to UV radiation, which can help outliers (e.g., users who have a history of burning more than average for their ITA). The answers can be obtained via user inputs on a phototherapy device (e.g., through a touchscreen on the device 100 of Fig. 1A) and/or inputs on a device in communication with the phototherapy device (e.g., inputs on the user's smartphone). In some embodiments, the process 200 skips block 208. For example, the process 200 can skip block 208 when a user is receiving treatments daily to avoid repeatedly prompting the user for answers.

At block 210, the process 200 includes determining a dosing protocol based on the calculated MED and/or the answers to the safety questions. The dosing protocol can include determining a power level for the UV emitters, a duration of the exposure, and/or the like to adjust the total UV radiation delivered to the user and/or the density of the UV radiation. In some embodiments, the dosing protocol is set to deliver less than the user's MED. For example, the dosing protocol can be set to deliver between about 05. MED and 0. about 95 MED, between about 0.6 MED and about 0.9 MED, or about 0.8 MED. In these examples, the dosing protocol can help reduce the chance that a user experiences a UV-induced pigmentation (e.g., a tan or burn) from the phototherapy device (e.g., when they have an outlier MED based on their ITA, when they also receive UV radiation from sun exposure outside of using the phototherapy device, and the like). Additionally, when the answers to the safety questions indicate that the user is on a medication that will reduce their ability to handle UV radiation, that the user has recently received a significant dose of UV radiation (e.g., from prolonged sun exposure), and/or that the user has a high propensity to burn, the dosing protocol can be set below the calculated MED. In some embodiments, the dosing protocol is translated directly from the MED to deliver UV radiation at a specified intensity and time such that the total UV exposure equates to the maximum UV radiation the user can undergo without experiencing an erythemal event (e.g., no burn, no significant skin coloration). In some embodiments, the dosing protocol is translated directly from the MED to deliver UV radiation at a specified intensity and time such that the total UV exposure equates to a level of UV radiation below the maximum amount without experiencing an erythemal event, while still providing significant vitamin D stimulation.

Because the process 200 is based largely on obtaining images of the treatment region and computations based on the obtained images, various steps of the process 200 can be executed instantly (or almost instantly). As a result, the primary bottlenecks for the process 200 are the speed at which the process 200 can obtain the images of the treatment region and/or the speed at which a user can provide answers to the safety questions. Thus, a user can have a streamlined experience with a phototherapy system implementing the process 200 to determine their dosing protocol. Further, because the process 200 can be implemented in its entirety relatively quickly (e.g., on the order of minutes), the process 200 can be implemented each time the user receives phototherapy treatment to quickly and accurately determine an appropriate dosing protocol that delivers their MED (or a dose close to their MED). That is, rather than ramping up from a known safe dose to their MED over a few weeks and/or requiring an irradiation test that takes at least 24 hours to provide results, the process 200 can calculate the user's MED, determine an appropriate dosing protocol, and allow a phototherapy device to provide treatment to the user the same day. The streamlined process can improve outcomes by more quickly providing treatment to new users, rapidly adjust for changes in the user's MED (e.g., as the user builds a suntan, loses a suntan, builds a tolerance to the treatment, and/or the like), and rapidly adjust for a user who misses treatments for an extended period (and thereby loses some tolerance).

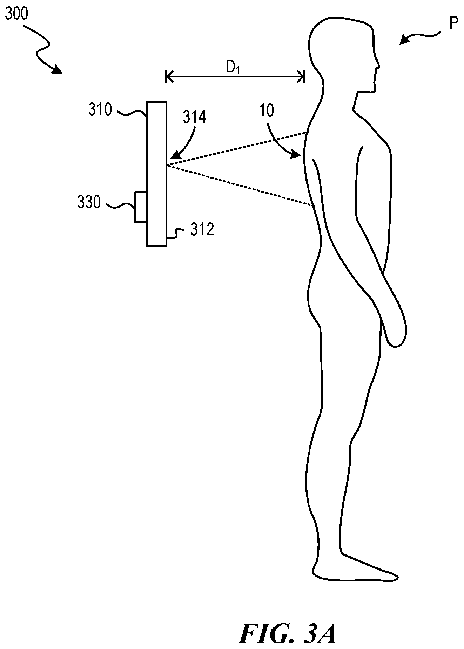

Fig. 3A is a partially schematic side view of a phototherapy system 300 while determining a dosing protocol for a user in accordance with some embodiments of the present technology. More specifically, Fig. 3A illustrates the phototherapy system 300 ("system 300") obtaining images of a treatment region of a user P according to block 202 of Fig. 2. As illustrated in Fig. 3A, the system 300 includes a phototherapy device 310 ("device 310") that can be generally similar to (or identical to) the phototherapy device 100 discussed above with reference to Figs. 1A and 1B. Further, as illustrated in Fig. 3A, the phototherapy device 310 includes an active surface 312 and a color testing component 314 positioned to image forward from the active surface 312. To determine their dosing protocol, the user P can stand in front of the active surface while the color testing component 314 takes one or more images of a testing region 10 (e.g., a portion of a treatment region). In the illustrated embodiment, the testing region 10 is on the back torso of the user P. However, it will be understood that the testing region 10 can be any other suitable portion of the user P that will receive the UV radiation (e.g., a user's front torso and/or the like).

In various embodiments, the color testing component 314 can image an area with a width (or diameter) between about 2.54 cm (1 inch) and about 38.1 cm (15 inches), between about 12.7cm (5 inches) and about 25.4cm (10 inches), or about 20.32 (8 inches). As discussed above, the larger areas images by the color testing component 314 can allow the system 300 (or another component communicably coupled thereto, such as a remote server) to determine an average ITA and/or average MED across the testing region 10. In turn, the average ITA and/or average MED can help calculate a more accurate dosing protocol for the user P across the entire treatment region. For example, a relatively small sample of the testing region 10 (e.g., less than 2.54cm (1 inch)) can inadvertently sample a region with a darker suntan than the majority of the testing region 10, and therefore a higher tolerance for UV radiation. In this example, the calculated MED will be higher than if the ITA and/or MED were averaged for a relatively large area (e.g., more than 6.452cm² (1 square inch), such as across an 51.613cm² (8 square inch) region). As a result, the user P would be exposed to a dose of UV radiation that is more likely to cause some level of erythema than if a larger sample were used.

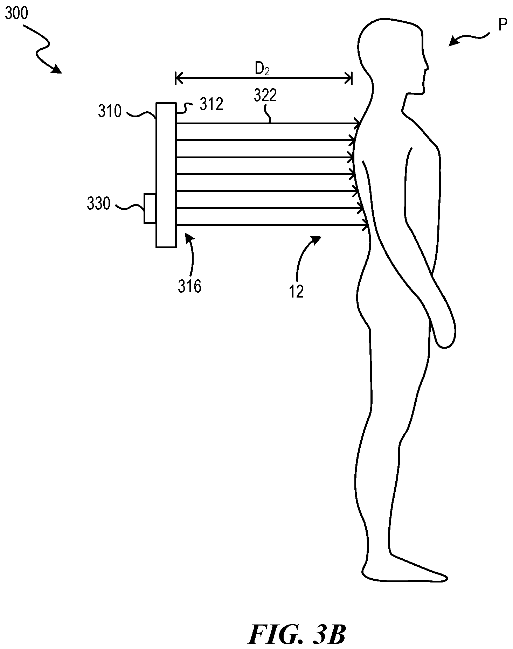

Fig. 3B is a partially schematic side view of the system 300 of Fig. 3A while operating according to a dosing protocol in accordance with some embodiments of the present technology. As illustrated in Fig. 3B, the device 310 includes one or more UV emitters 316 (e.g., an array of LED components and/or other optical elements) positioned to emit UV radiation 322 forward from the active surface 312. Accordingly, when the user P stands in front of the device 310, the UV emitters 316 deliver the UV radiation 322 to a treatment region 12 on the back torso of the user P (or any other suitable location). As further illustrated in Fig. 3A, the system 300 includes a controller 330 operably coupled to the color testing component 314 and the UV emitters 316 to control operation of the system 300. Purely by way of example, the controller 330 can implement a process generally similar (or identical) to the process 200 discussed above with reference to Fig. 2 to determine a dosing protocol for the user P, then cause the system 300 to deliver UV radiation in accordance with the dosing protocol.

In some embodiments, as illustrated in Figs. 3A and 3B, the user P can stand a first distance D₁ (Fig. 3A) from the device 310 while determining their dosing protocol (e.g., during a testing phase), then stand a second distance D₂ from the device 310 while receiving the dose of UV radiation. In some embodiments, the first distance D₁ is less than the second distance D₂. In such embodiments, for example, the user P stands a relatively short distance from the device 310 during the testing phase to help increase the accuracy of the measurements by reducing the impact of ambient light. They then move away from the device 310 during the dosing phase to help improve the safety of the device 310 (e.g., standing too close can cause overexposure). In some embodiments, however, the first distance D₁ is equal to (or generally equal to) the second distance D₂, allowing the user to stand in one location while the phototherapy system 300 determines an appropriate dosing protocol and delivers a dose of UV radiation according to the dosing protocol.

### Selected Embodiments of Systems and Methods for Dose-Testing a User's Skin

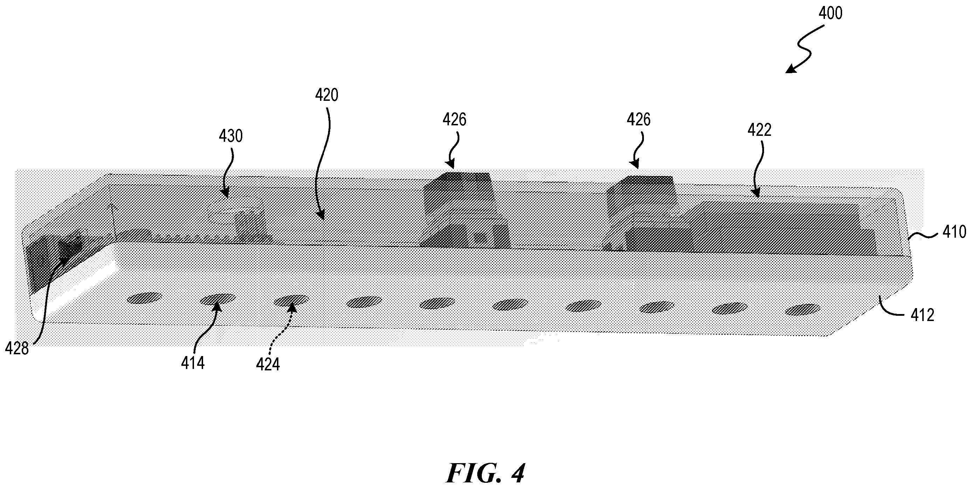

Fig. 4 is an isometric view of a testing component 400 for determining a dosing protocol for a phototherapy system configured in accordance with some embodiments of the present technology. In some embodiments, the testing component 400 is communicatively coupled to a phototherapy device, such as the device 100 discussed above with reference to Fig. 1A. In other embodiments, the testing component 400 is independent, allowing a user (or medical professional) to test a user's MED then independently provide results to a phototherapy device.

In the illustrated embodiment, the testing component 400 includes a housing 410 and active electronics 420 contained within the housing 410. The housing includes a testing surface 412 (e.g., an active surface, a lower surface, and/or the like) that includes a plurality of openings 414. The active electronics 420 include an input/output (I/O) component 422, a plurality of UV emitters 424 (e.g., LEDs) aligned with the plurality of openings 414, one or more status lights 426 (two illustrated in Fig. 4), and a power port 428 for connection to a power cord (e.g., to charge and/or power the testing component 400). The I/O component 422 can include a touchscreen, one or more input buttons, one or more rotatable components (e.g., a knob, rotary switch, and/or the like), switches, a display, and/or any other suitable component. The inputs allow a user (or medical professional) to turn the testing component 400 on, set parameters for a testing dose (e.g., minimum dose, maximum dose, steps between each dose, and/or the like), start delivering a dose, stop delivering a dose, select and estimated skin type, select an estimated MED, and/or the like. For example, a user can turn the testing component on, and input their estimated ITA, skin type, and/or MED. The I/O component 422 can then use the inputs to determine a testing range (e.g., -25% of MED to +25% of MED) and set a testing protocol for the plurality of UV emitters 424.

As discussed in more detail below, the test protocol causes each of the plurality of UV emitters 424 to deliver a different dose of UV radiation that can then be used to accurately determine an appropriate dosing protocol. For example, the plurality of UV emitters 424 can deliver a percentage of the user's estimated MED in steps of 5%. In this example, from left to right, the plurality of UV emitters 424 can deliver 75% of the user's estimated MED, 80% of the user's estimated MED, 85% of the user's estimated MED, 90% of the user's estimated MED, 95% of the user's estimated MED, the user's estimated MED, 105% of the user's estimated MED, 110% of the user's estimated MED, 115% of the user's estimated MED, and 120% of the user's estimated MED. In various other examples, the steps can be 1%, 2, 3%, 4%, 10%, and/or any other suitable step. Additionally, or alternatively, the maximum dose can be shifted such that one, two, three, and/or any other suitable number of the plurality of UV emitters 424 delivers a dose over the user's estimated MED.

As further illustrated in Fig. 4, the testing component 400 can include a calibration port 430. The calibration port 430 can provide access to the active electronics 420 to an external device (e.g., a computer, smart device, and/or the like) to test and/or calibrate the active electronics 420, install one or more updates, and/or the like. Additionally, or alternatively, the calibration port 430 can provide a sample point for ambient conditions (e.g., humidity, light, and/or the like), allowing the testing component 400 to adjust a testing dose based on the ambient conditions.

In some embodiments, the testing component 400 includes a color measurement device (e.g., a colorimeter) coupled to the active electronics 420. For example, the color measurement device can be aligned with one of the plurality of openings 414 to image the user's skin. The image can be used to obtain an estimate of the user's ITA, skin type, and/or MED. In turn, as discussed above, the estimate can be used to set a range (and steps) for doses delivered by the testing component 400. In some such embodiments, because the color measurement device can provide a relatively accurate estimate of the user's ITA, skin type, and/or MED, the range of doses delivered by the testing component 400 can be smaller. As a result, the accuracy of results from the testing component 400 can be improved, allowing the appropriate dose for the user to be more accurately determined.

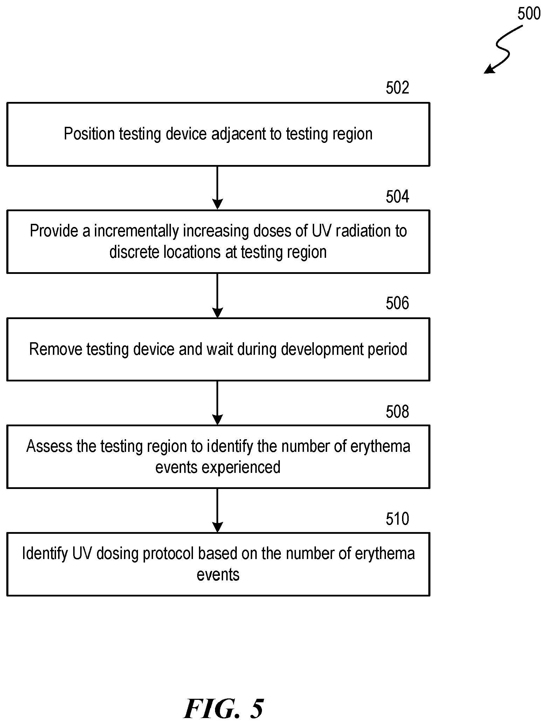

Fig. 5 is a flow diagram of a process 500 for determining a dosing protocol using a testing component in accordance with some embodiments of the present technology. The process 500 can be implemented in conjunction with a testing component 400 of the type discussed above with reference to Fig. 4 by a user, a medical professional, and/or a phototherapy system coupled to the testing device.

The process 500 begins at block 502 by positioning the testing device adjacent to a testing region on the user. For example, the active surface 412 of the housing 410 (Fig. 4) can be placed in contact with a user's skin in a selected testing region. The testing region can be the region of the user that will be treated (e.g., the user's back torso) and/or an easily accessible region (e.g., the user's wrist).

At block 504, the process 500 provides incrementally increasing doses of UV radiation to discrete locations at the testing region. For example, each of the plurality of UV emitters 424 (Fig. 4) can deliver a different dose of UV radiation. To do so, for example, the process 500 can vary the power level of the UV emitters delivering UV radiation and/or vary the exposure duration. In some embodiments, the range of the incrementally increasing doses allows the process 500 to determine an appropriate dose for the user with no information on the user (e.g., ranging from doses for the most UV-sensitive users to doses for the least UV-sensitive users). In such embodiments, the process 500 requires no input from the user to determine an appropriate dose. In some embodiments, the process 500 uses estimates and/or measures of a user's ITA, skin type, and/or MED before beginning the process 500 to determine a starting point and/or a range for the doses. For example, the process 500 can include obtaining images (and/or other measurements) of the user's skin, calculating an ITA in the testing region, calculating an estimated MED from the ITA, and multiplying the estimated MED by a fraction to identify a starting point for the doses. In such embodiments, the process 500 can determine the appropriate dose at a higher level of granularity and/or avoid providing doses significantly over the user's appropriate level.

At block 506, the process 500 includes removing the testing device and waiting for a predetermined development period. The development period is the time the user's skin takes to develop color (if any) in response to the UV doses. In general, it takes bout 24 hours before all erythema (or suntanning) in response to the UV doses fully develops. However, in various embodiments, the predetermined development period can be between 12 and 48 hours, allowing the process 500 to be implemented at times that are compatible with the user's schedule.

At block 508, the process 500 includes assessing the testing region to identify the number of erythema events that the user experienced and/or the last dose that did not cause erythema. The assessment includes looking for any perceptible redness at the discrete locations in the testing region. In some embodiments, the assessment is completed by the user and/or another person e.g., a medical professional). In some embodiments, the assessment is completed by imaging the testing region and processing the image to detect discrete erythema events.

At block 510, the process 500 includes identifying a UV dosing protocol based on the results of block 508. For example, the dosing protocol can be configured to deliver a dose equal to (or generally equal to) the last dose that did not cause an erythema event. In some embodiments, the dosing protocol can be based on the number of erythema events that occurred. For example, when no information on the user was used to set the range of doses, the process 500 can extrapolate from the number of erythema events the user experienced to an estimated MED for the user. The process can then set the dosing protocol to deliver the estimated MED (or a percentage of the estimated MED).

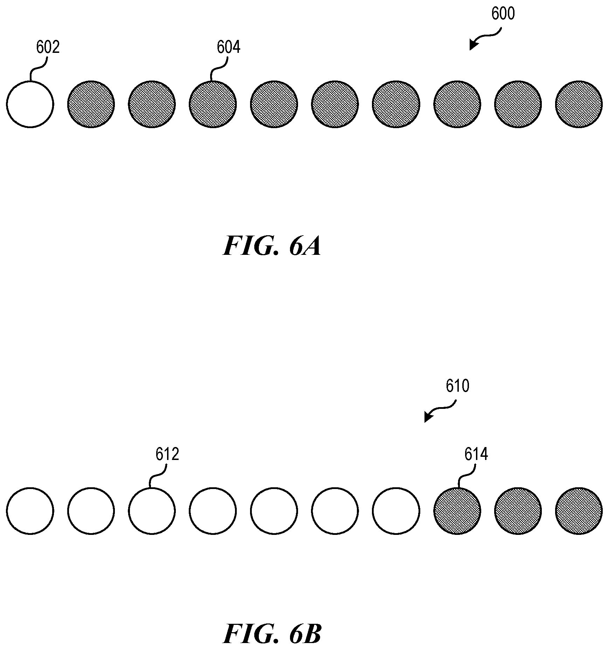

Figs. 6A and 6B illustrate results 600, 610 of block 506 of the process of Fig. 5 for different users in accordance with some embodiments of the present technology. In the illustrated embodiment, the same doses of UV radiation were delivered at each discrete location in the testing region. The results 600 illustrated in Fig. 6A include a single non-effected location 602 and nine effected locations 604. The results 600 indicate that the corresponding user is relatively sensitive to UV radiation. As a result, the process 500 could set the dosing protocol to deliver a relatively low amount of UV radiation during phototherapy treatments. In contrast, the results 610 illustrated in Fig. 6B include seven non-effected locations 612 and three effected locations 614. The results 610 indicate that the corresponding user has an above-average tolerance to UV radiation. As a result, the process 500 could set the dosing protocol to deliver mid-high amounts of UV radiation during phototherapy treatments.

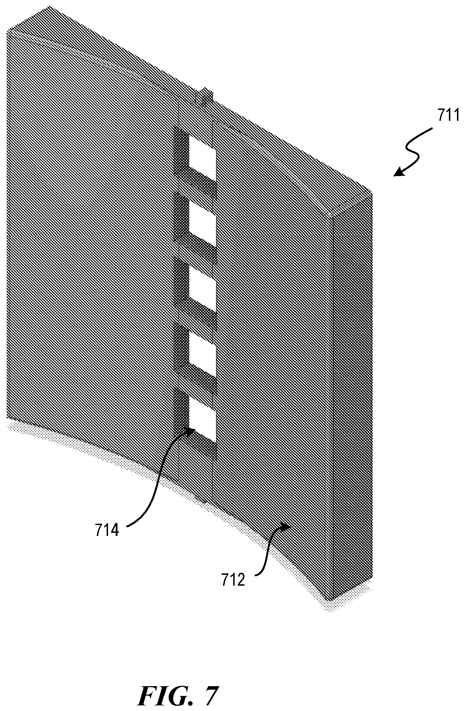

Fig. 7 is an isometric view of a skin-interfacing component 711 for a testing component of a phototherapy system configured in accordance with some embodiments of the present technology. The skin-interfacing component 711 can define a portion of and/or be positioned on an active surface of the testing component 400 of Fig. 4. In the illustrated embodiment, the skin-interfacing component 711 includes a skin-contacting surface 712 (also referred to as an outermost surface, a first surface, an underside, and the like) and a plurality of openings 714 that extend through the skin-contacting surface 712. The plurality of openings 714 provide pathways through the skin-interfacing component 711 for UV emitters and/or color testing components in the testing component to access a user's skin. In the illustrated embodiment, the skin-interfacing component 711 includes five openings 714 that align with corresponding UV emitters and/or color testing components. In some embodiments, the skin-interfacing component 711 has fewer than five openings 714 or more than five openings 714 that align with the corresponding number of UV emitters and/or color testing components.

As further illustrated in Fig. 7, the skin-contacting surface 712 can have a curved profile (e.g., define a rounded surface) that allows the surface 712 of the skin-interfacing component 711 to lay flush or substantially flush against the user's skin at a testing region (e.g., on the wrist, side of the user's torso, and/or the like). As a result, the curved profile can help block ambient light during the tests and/or help contain UV radiation to the discrete locations accessed through the plurality of openings 714 (e.g., thereby preventing accidental exposures from UV radiation leaking out).

### Conclusion

From the foregoing, it will be appreciated that specific embodiments of the technology have been described herein for purposes of illustration, but well-known structures and functions have not been shown or described in detail to avoid unnecessarily obscuring the description of the embodiments of the technology. To the extent any material incorporated herein by reference conflicts with the present disclosure, the present disclosure controls. Where the context permits, singular or plural terms may also include the plural or singular term, respectively. Moreover, unless the word "or" is expressly limited to mean only a single item exclusive from the other items in reference to a list of two or more items, then the use of "or" in such a list is to be interpreted as including (a) any single item in the list, (b) all of the items in the list, or (c) any combination of the items in the list. Furthermore, as used herein, the phrase "and/or" as in "A and/or B" refers to A alone, B alone, and both A and B. Additionally, the terms "comprising," "including," "having," and "with" are used throughout to mean including at least the recited feature(s) such that any greater number of the same features and/or additional types of other features are not precluded. Further, the terms "approximately" and "about" are used herein to mean within at least within 10% of a given value or limit. Purely by way of example, an approximate ratio means within a 10% of the given ratio.

Several implementations of the disclosed technology are described above in reference to the figures. The computing devices on which the described technology may be implemented can include one or more central processing units, memory, input devices (e.g., keyboard and pointing devices), output devices (e.g., display devices), storage devices (e.g., disk drives), and network devices (e.g., network interfaces). The memory and storage devices are computer-readable storage media that can store instructions that implement at least portions of the described technology. In addition, the data structures and message structures can be stored or transmitted via a data transmission medium, such as a signal on a communications link. Various communications links can be used, such as the Internet, a local area network, a wide area network, or a point-to-point dial-up connection. Thus, computer-readable media can comprise computer-readable storage media (e.g., "non-transitory" media) and computer-readable transmission media.

From the foregoing, it will also be appreciated that various modifications may be made without deviating from the disclosure or the technology as set forth in the claims. For example, one of ordinary skill in the art will understand that various components of the technology can be further divided into subcomponents, or that various components and functions of the technology may be combined and integrated. In addition, certain aspects of the technology described in the context of particular embodiments may also be combined or eliminated in other embodiments.

Furthermore, although advantages associated with certain embodiments of the technology have been described in the context of those embodiments, other embodiments may also exhibit such advantages, and not all embodiments need necessarily exhibit such advantages to fall within the scope of the technology as set forth in the claims. Accordingly, the disclosure and associated technology can encompass other embodiments not expressly shown or described herein.

## Claims

1. A method for determining a dosing protocol for irradiation of a user's skin (200), the method comprising:
obtaining one or more images of a treatment region of the user's skin (202) from a color testing component (140);
determining, via a controller (120) of a phototherapy device, an average individual topography angle, ITA, for the user's skin across the treatment region (10) based on the one or more images;
determining, via the controller (120), an estimated minimal erythema dose, MED, for the treatment region based on the average ITA across the treatment region; and
creating, via controller, the dosing protocol based on the estimated MED for the treatment region, wherein the dosing protocol comprises control settings for one or more ultraviolet, UV, emitters (130) in the phototherapy device (100) to deliver a dose of UV radiation to the user's skin during an individual treatment session that is below the estimated MED for the user's skin.

2. The method (200) of claim 1, wherein obtaining the one or more images of the treatment region comprises:
emitting light with a predetermined composition of wavelengths; and
measuring a reflection back from the treatment region using an imaging sensor.

3. The method (200) of claim 1, wherein obtaining the one or more images of the treatment region comprises:
capturing one or more pre-filter images of the treatment region;
capturing one or more images of a calibration component having a known color composition;
determining a corrective filter for the one or more pre-filter images of the treatment region based on corrections needed for the one or more images of the calibration component and the known color composition; and
applying the corrective filter to each of the one or more pre-filter images of the treatment region.

4. The method (200) of claim 1, wherein obtaining the one or more images of the treatment region from the color testing component comprises receiving the one or more images from an imaging sensor on the phototherapy device (100).

5. The method (200) of claim 1, wherein determining an average ITA for the user's skin across the treatment region comprises:
converting the one or more images of the treatment region to an L*a*b* format; and
for each individual image, calculating an individual ITA of the individual image from the L*a*b* format.

6. The method (200) of claim 5, wherein the individual ITA is calculated using: $ITA = \frac{arctan \left(\frac{L * - 50}{b *}\right) \times 180}{π}$ .

7. The method (200) of claim 5, wherein determining an average ITA for the user's skin across the treatment region further comprises, for each individual image averaging L*a*b* data in the individual image before calculating the individual ITA of the individual image.

8. The method (200) of claim 5, wherein determining an average ITA for the user's skin across the treatment region further comprises determining, via the controller (120), the average ITA based on an average of the individual ITAs from each of the individual images.

9. The method (200) of claim 1, wherein the estimated MED is calculated using *MED =* (*ITA*² × 0.051) *-* (*ITA* × 10.718) + 629.32.

10. The method (200) of claim 1, further comprising receiving, via a user input device, user inputs associated with an answer to each of one or more secondary questions, wherein the one or more secondary questions relate to factors affecting an actual MED for the user's skin across the treatment region.

11. The method (200) of claim 10, wherein the one or more secondary questions comprise one or more of:
whether the user is taking a medication that affects MED;
whether the user has recently been exposed to sunlight and/or other UV radiation; and/or
whether the user has a known susceptibility to UV radiation.

12. The method (200) of claim 10 wherein identifying the dosing protocol comprises identifying a power level for one or more UV emitters in a phototherapy device and a duration of an exposure during treatment to adjust an intensity and a total amount of UV radiation delivered to the treatment region of the user's skin.

13. The method (200) of claim 10 wherein identifying the dosing protocol comprises identifying treatment parameters to deliver a total amount of UV radiation user of about 0.8 of the estimated MED.

14. A phototherapy system (300), comprising:
a phototherapy device (100); 310) having an active surface (312) with a plurality of light emitting diodes, LEDs, (130) positioned to emit ultraviolet, UV, radiation forward from the active surface (312) during operation of the phototherapy system (300);
a color testing component (140; 314) positioned to obtain one or more images of a treatment region (10) of a user's skin when the user stands forward from the active surface (312); and
a controller (120; 330) operably coupled to the plurality of LEDs (130) and the color testing component (140; 314), wherein the controller (120; 330) comprises a processor and a memory storing instructions that, when executed by the processor cause the controller to:
receive one or more images of the treatment region of the user's skin from the color testing component (140; 314) when the user stands a predetermined distance from the active surface (312);
determine an average individual topography angle, ITA,
of the user's skin across the treatment region based on the one or more images;
determine an estimated minimal erythema dose, MED, for the treatment region based on the average ITA across the treatment region;
provide, to the phototherapy device, a dosing protocol based on the estimated MED for the treatment region, wherein the dosing protocol comprises control settings for the plurality of LEDs (130) to deliver a dose of UV radiation to the user's skin during an individual treatment session that is below the estimated MED; and
cause the phototherapy device to operate the plurality of LEDs (130) in accordance with the dosing protocol.

15. The phototherapy system (300) of claim 14, wherein the predetermined distance is between about 12.7 cm and about 25.4cm away from the active surface.

16. The phototherapy system (300) of claim 14, wherein the average ITA corresponds to an average of ITA values across a portion of the treatment region having an area greater than 6.45 cm².

17. The phototherapy system (300) of claim 14, wherein:
the phototherapy device (100) comprises:
an upper panel (114) having a first portion of the active surface; and
a lower panel (116) coupled to the upper panel (114) by a hinge (118) and having a second portion of the active surface; and
the phototherapy device (100) is movable between a collapsed position with the second portion of the active surface directed toward the first portion of the active surface and a deployed position with the first and second portions of the active surface directed forward.

## Patentansprüche

1. Verfahren zur Bestimmung eines Dosierprotokolls zur Bestrahlung einer Haut eines Benutzers (200), wobei das Verfahren umfasst:
Beziehen eines oder mehrerer Bilder einer Behandlungsregion der Haut des Benutzers (202) von einer Farbtestkomponente (140);
Bestimmen eines durchschnittlichen einzelnen Topographiewinkels, ITA, für die Haut des Benutzers über die Behandlungsregion (10) auf der Basis des einen oder der mehreren Bilder mittels eines Controllers (120) einer Phototherapievorrichtung;
Bestimmen einer geschätzten minimalen Erythemdosis, MED, für die Behandlungsregion auf der Basis des durchschnittlichen ITA über die Behandlungsregion mittels des Controllers (120); und
Erzeugen des Dosierprotokolls auf der Basis der geschätzten MED für die Behandlungsregion mittels des Controllers, wobei das Dosierprotokoll Steuereinstellungen für einen oder mehrere Ultraviolett-Emitter, UV-Emitter (130), in der Phototherapievorrichtung (100) zum Abgeben einer Dosis von UV-Strahlung an die Haut des Benutzers während einer einzelnen Behandlungssitzung umfasst, die unterhalb der geschätzten MED für die Haut des Benutzers liegt.

2. Verfahren (200) nach Anspruch 1, wobei das Beziehen des einen oder der mehreren Bilder der Behandlungsregion umfasst:
Emittieren von Licht mit einer vorbestimmten Zusammensetzung von Wellenlängen;
und
Messen einer Rückreflexion von der Behandlungsregion unter Verwendung eines Bildgebungssensors.

3. Verfahren (200) nach Anspruch 1, wobei das Beziehen des einen oder der mehreren Bilder der Behandlungsregion umfasst:
Aufnehmen eines oder mehrerer Vor-Filter-Bilder der Behandlungsregion;
Aufnehmen eines oder mehrerer Bilder einer Kalibrierkomponente mit einer bekannten Farbzusammensetzung;
Bestimmen eines Korrekturfilters für das eine oder die mehreren Vor-Filter-Bilder der Behandlungsregion auf der Basis von Korrekturen, die für das eine oder die mehreren Bilder der Kalibrierkomponente erforderlich sind, und der bekannten Farbzusammensetzung; und
Anwenden des Korrekturfilters auf jedes von dem einen oder den mehreren Vor-Filter-Bildern der Behandlungsregion.

4. Verfahren (200) nach Anspruch 1, wobei das Beziehen des einen oder der mehreren Bilder der Behandlungsregion von der Farbtestkomponente ein Empfangen des einen oder der mehreren Bilder von einem Bildgebungssensor an der Phototherapievorrichtung (100) umfasst.

5. Verfahren (200) nach Anspruch 1, wobei das Bestimmen eines durchschnittlichen ITA für die Haut des Benutzers über die Behandlungsregion umfasst:
Umwandeln des einen oder der mehreren Bilder der Behandlungsregion in ein L*a*b*-Format; und
für jedes einzelne Bild Berechnen eines einzelnen ITA des einzelnen Bilds aus dem L*a*b*-Format.

6. Verfahren (200) nach Anspruch 5, wobei der einzelne ITA berechnet wird unter Verwendung von: $ITA = \frac{arctan \left(\frac{L * - 50}{b *}\right) \times 180}{π}$.

7. Verfahren (200) nach Anspruch 5, wobei das Bestimmen eines durchschnittlichen ITA für die Haut des Benutzers über die Behandlungsregion ferner für jedes einzelne Bild ein Mitteln von L*a*b*-Daten in dem einzelnen Bild vor dem Berechnen des einzelnen ITA des einzelnen Bilds umfasst.

8. Verfahren (200) nach Anspruch 5, wobei das Bestimmen eines durchschnittlichen ITA für die Haut des Benutzers über die Behandlungsregion ferner ein Bestimmen des durchschnittlichen ITA auf der Basis eines Durchschnitts der einzelnen ITA aus jedem der einzelnen Bilder mittels des Controllers (120) umfasst.

9. Verfahren (200) nach Anspruch 1, wobei die geschätzte MED unter Verwendung von *MED =* (*ITA²* x 0,051) - (*ITA* x 10,718) + 629,32 berechnet wird.

10. Verfahren (200) nach Anspruch 1, ferner umfassend ein Empfangen von Benutzereingaben, die mit einer Antwort auf jede von einer oder mehreren sekundären Fragen assoziiert sind, mittels einer Benutzereingabevorrichtung, wobei die eine oder die mehreren sekundären Fragen Faktoren betreffen, die sich auf eine tatsächliche MED für die Haut des Benutzers über die Behandlungsregion auswirken.

11. Verfahren (200) nach Anspruch 10, wobei die eine oder die mehreren sekundären Fragen eine oder mehrere der folgenden umfassen:
ob der Benutzer ein Medikament einnimmt, das sich auf eine MED auswirkt;
ob der Benutzer vor kurzem gegenüber Sonnenlicht und/oder einer anderen UV-Strahlung exponiert war; und/oder
ob der Benutzer eine bekannte Empfindlichkeit für UV-Strahlung hat.

12. Verfahren (200) nach Anspruch 10, wobei das Identifizieren des Dosierprotokolls ein Identifizieren eines Leistungspegels für einen oder mehrere UV-Emitter in einer Phototherapievorrichtung und einer Dauer einer Exposition während einer Behandlung umfasst, um eine Intensität und eine Gesamtmenge von UV-Strahlung, die an die Behandlungsregion der Haut des Benutzers abgegeben wird, zu justieren.

13. Verfahren (200) nach Anspruch 10, wobei das Identifizieren des Dosierprotokolls ein Identifizieren von Behandlungsparametern zum Abgeben einer Gesamtmenge von UV-Strahlung von etwa 0,8 der geschätzten MED an einen Benutzer umfasst.

14. Phototherapiesystem (300), umfassend:
eine Phototherapievorrichtung (100; 310), aufweisend eine aktive Oberfläche (312) mit einer Vielzahl von Leuchtdioden, LED (130), die positioniert ist, um Ultraviolettstrahlung, UV-Strahlung, von der aktiven Oberfläche (312) während eines Betriebs des Phototherapiesystems (300) nach vorne zu emittieren;
eine Farbtestkomponente (140; 314), die positioniert ist, um ein oder mehrere Bilder einer Behandlungsregion (10) einer Haut eines Benutzers zu beziehen, wenn der Benutzer nach vorne von der aktiven Oberfläche (312) steht; und
einen Controller (120; 330), der betriebsfähig an die Vielzahl von LED (130) und die Farbtestkomponente (140; 314) gekoppelt ist, wobei der Controller (120; 330) einen Prozessor und einen Speicher, der Anweisungen speichert, umfasst, die bei Ausführung durch den Prozessor den Controller veranlassen zum:
Empfangen eines oder mehrerer Bilder der Behandlungsregion der Haut des Benutzers von der Farbtestkomponente (140; 314), wenn der Benutzer in einer vorbestimmten Entfernung von der aktiven Oberfläche (312) steht;
Bestimmen eines durchschnittlichen einzelnen Topographiewinkels, ITA, der Haut des Benutzers über die Behandlungsregion auf der Basis des einen oder der mehreren Bilder;
Bestimmen einer geschätzten minimalen Erythemdosis, MED, für die Behandlungsregion auf der Basis des durchschnittlichen ITA über die Behandlungsregion;
Bereitstellen eines Dosierprotokolls auf der Basis der geschätzten MED für die Behandlungsregion an die Phototherapievorrichtung, wobei das Dosierprotokoll Steuereinstellungen für die Vielzahl von LED (130) zum Abgeben einer Dosis von UV-Strahlung an die Haut des Benutzers während einer einzelnen Behandlungssitzung umfasst, die unterhalb der geschätzten MED liegt; und
Veranlassen der Phototherapievorrichtung, die Vielzahl von LED (130) gemäß dem Dosierprotokoll zu betreiben.

15. Phototherapiesystem (300) nach Anspruch 14, wobei die vorbestimmte Entfernung zwischen etwa 12,7 cm und etwa 25,4 cm von der aktiven Oberfläche weg ist.

16. Phototherapiesystem (300) nach Anspruch 14, wobei der durchschnittliche ITA einem Durchschnitt von ITA-Werten über einen Teil der Behandlungsregion entspricht, der eine Fläche von mehr als 6,45 cm² aufweist.

17. Phototherapiesystem (300) nach Anspruch 14, wobei:
die Phototherapievorrichtung (100) umfasst:
ein oberes Paneel (114), der einen ersten Teil der aktiven Oberfläche aufweist; und
ein unteres Paneel (116), das durch ein Scharnier (118) an das obere Paneel (114) gekoppelt ist und einen zweiten Teil der aktiven Oberfläche aufweist; und
wobei die Phototherapievorrichtung (100) zwischen einer zusammengelegten Position, wobei der zweite Teil der aktiven Oberfläche zu dem ersten Teil der aktiven Oberfläche hin ausgerichtet ist, und einer entfalteten Position, wobei der erste und der zweite Teil der aktiven Oberfläche nach vorne ausgerichtet sind, bewegbar ist.

## Revendications

1. Procédé pour déterminer un protocole de dosage pour l'irradiation de la peau d'un utilisateur (200), le procédé comprenant :
l'obtention d'une ou plusieurs images d'une région de traitement de la peau de l'utilisateur (202) à partir d'un composant de test de couleur (140) ;
la détermination, par l'intermédiaire d'un dispositif de commande (120) d'un dispositif de photothérapie, d'un angle de topographie individuel moyen, ITA, pour la peau de l'utilisateur sur l'ensemble de la région de traitement (10) sur la base des unes ou plusieurs images ;
la détermination, par l'intermédiaire du dispositif de commande (120), d'une dose érythémateuse minimale, MED, estimée pour la région de traitement sur la base de l'ITA moyen sur l'ensemble de la région de traitement ; et
la création, par l'intermédiaire du dispositif de commande, du protocole de dosage sur la base de la MED estimée pour la région de traitement, le protocole de dosage comprenant des paramètres de commande pour un ou plusieurs émetteurs d'ultraviolets, UV, (130) dans le dispositif de photothérapie (100) pour administrer à la peau de l'utilisateur, au cours d'une séance de traitement individuelle, une dose de rayonnement UV qui est inférieure à la MED estimée pour la peau de l'utilisateur.

2. Procédé (200) selon la revendication 1, dans lequel l'obtention des unes ou plusieurs images de la région de traitement comprend :
l'émission de lumière ayant une composition prédéterminée en longueurs d'onde ; et
la mesure d'une réflexion en retour de la région de traitement à l'aide d'un capteur d'imagerie.

3. Procédé (200) selon la revendication 1, dans lequel l'obtention des unes ou plusieurs images de la région de traitement comprend :
la capture d'une ou plusieurs images avant filtrage de la région de traitement ;
la capture d'une ou plusieurs images d'un composant d'étalonnage présentant une composition chromatique connue ;
la détermination d'un filtre correctif pour les une ou plusieurs images avant filtrage de la région de traitement sur la base des corrections nécessaires pour les une ou plusieurs images du composant d'étalonnage et de la composition chromatique connue ; et
l'application du filtre correctif à chacune des unes ou plusieurs images avant filtrage de la région de traitement.

4. Procédé (200) selon la revendication 1, dans lequel l'obtention des unes ou plusieurs images de la région de traitement à partir du composant de test de couleur comprend la réception des unes ou plusieurs images provenant d'un capteur d'imagerie sur le dispositif de photothérapie (100).

5. Procédé (200) selon la revendication 1, dans lequel la détermination d'un ITA moyen pour la peau de l'utilisateur sur l'ensemble de la région de traitement comprend :
la conversion des unes ou plusieurs images de la région de traitement en un format L*a*b* ; et
pour chaque image individuelle, le calcul d'un ITA individuel de l'image individuelle à partir du format L*a*b*.

6. Procédé (200) selon la revendication 5, dans lequel l'ITA individuel est calculé à l'aide de la formule $ITA = \frac{arctan \left(\frac{L * - 50}{b *}\right) \times 180}{π}$.

7. Procédé (200) selon la revendication 5, dans lequel la détermination d'un ITA moyen pour la peau de l'utilisateur sur l'ensemble de la région de traitement comprend en outre, pour chaque image individuelle, le calcul de la moyenne des données L*a*b* dans l'image individuelle avant le calcul de l'ITA individuel de l'image individuelle.

8. Procédé (200) selon la revendication 5, dans lequel la détermination d'un ITA moyen pour la peau de l'utilisateur sur l'ensemble de la région de traitement comprend en outre la détermination, par l'intermédiaire du dispositif de commande (120), de l'ITA moyen sur la base d'une moyenne des ITA individuels de chacune des images individuelles.

9. Procédé (200) selon la revendication 1, dans lequel la MED estimée est calculée à l'aide de la formule *MED* = (*ITA*² × 0,051) - (*ITA* × 10,718) + 629,32.

10. Procédé (200) selon la revendication 1, comprenant en outre la réception, par l'intermédiaire d'un dispositif de saisie utilisateur, de saisies utilisateur associées à une réponse à chacune d'une ou plusieurs questions secondaires, dans lequel les une ou plusieurs questions secondaires concernent des facteurs affectant une MED réelle pour la peau de l'utilisateur sur l'ensemble de la région de traitement.

11. Procédé (200) selon la revendication 10, dans lequel les une ou plusieurs questions secondaires comprennent une ou plusieurs de :
la question de savoir si l'utilisateur prend un médicament qui affecte la MED ;
la question de savoir si l'utilisateur a récemment été exposé à la lumière solaire et/ou à un autre rayonnement UV ; et/ou
la question de savoir si l'utilisateur présente une sensibilité connue au rayonnement UV.

12. Procédé (200) selon la revendication 10, dans lequel l'identification du protocole de dosage comprend l'identification d'un niveau de puissance pour un ou plusieurs émetteurs UV dans un dispositif de photothérapie et d'une durée d'exposition au cours du traitement pour régler une intensité et une quantité totale de rayonnement UV administré à la région de traitement de la peau de l'utilisateur.

13. Procédé (200) selon la revendication 10, dans lequel l'identification du protocole de dosage comprend l'identification de paramètres de traitement pour administrer à l'utilisateur une quantité totale de rayonnement UV d'environ 0,8 fois la MED estimée.

14. Système de photothérapie (300), comprenant :
un dispositif de photothérapie (100 ; 310) présentant une surface active (312) comportant une pluralité de diodes électroluminescentes, DEL, (130) positionnées pour émettre un rayonnement ultraviolet, UV, vers l'avant à partir de la surface active (312) pendant le fonctionnement du système de photothérapie (300) ;
un composant de test de couleur (140 ; 314) positionné pour obtenir une ou plusieurs images d'une région de traitement (10) de la peau d'un utilisateur lorsque l'utilisateur se tient devant la surface active (312) ; et
un dispositif de commande (120 ; 330) fonctionnellement couplé à la pluralité de DEL (130) et au composant de test de couleur (140 ; 314), le dispositif de commande (120 ; 330) comprenant un processeur et une mémoire dans laquelle sont enregistrées des instructions qui, lorsqu'elles sont exécutées par le processeur, amènent le dispositif de commande à :
recevoir une ou plusieurs images de la région de traitement de la peau de l'utilisateur à partir du composant de test de couleur (140 ; 314) lorsque l'utilisateur se tient à une distance prédéterminée de la surface active (312) ;
déterminer un angle de topographie individuel moyen, ITA, de la peau de l'utilisateur sur l'ensemble de la région de traitement sur la base des unes ou plusieurs images ;
déterminer une dose érythémateuse minimale, MED, estimée pour la région de traitement sur la base de l'ITA moyen sur l'ensemble de la région de traitement ;
fournir, au dispositif de photothérapie, un protocole de dosage sur la base de la MED estimée pour la région de traitement, le protocole de dosage comprenant des paramètres de commande pour la pluralité de DEL (130) pour administrer à la peau de l'utilisateur, au cours d'une séance de traitement individuelle, une dose de rayonnement UV qui est inférieure à la MED estimée ; et
amener le dispositif de photothérapie à faire fonctionner la pluralité de DEL (130) conformément au protocole de dosage.

15. Système de photothérapie (300) selon la revendication 14, dans lequel la distance prédéterminée par rapport à la surface active est comprise entre environ 12,7 cm et environ 25,4 cm.

16. Système de photothérapie (300) selon la revendication 14, dans lequel l'ITA moyen correspond à une moyenne de valeurs d'ITA sur l'ensemble d'une partie de la région de traitement ayant une aire supérieure à 6,45 cm².

17. Système de photothérapie (300) selon la revendication 14, dans lequel :
le dispositif de photothérapie (100) comprend :
un panneau supérieur (114) comportant une première partie de la surface active ; et
un panneau inférieur (116) couplé au panneau supérieur (114) par une charnière (118) et comportant une deuxième partie de la surface active ; et
le dispositif de photothérapie (100) peut être déplacé entre une position repliée dans laquelle la deuxième partie de la surface active est orientée vers la première partie de la surface active et une position déployée dans laquelle les première et deuxième parties de la surface active sont orientées vers l'avant.
